(19) Europäisches Patentamt
European Patent Office
Office européen des brevets

(11) Publication number: **0 194 599**
**A2**

# (12) EUROPEAN PATENT APPLICATION

(21) Application number: 86103029.4

(22) Date of filing: 07.03.86

(51) Int. Cl.[4]: C 07 D 215/22
A 01 N 43/42

(30) Priority: 14.03.85 JP 49275/85
08.02.86 JP 24945/86

(43) Date of publication of application:
17.09.86 Bulletin 86/38

(84) Designated Contracting States:
BE CH DE FR GB IT LI NL

(71) Applicant: NISSAN CHEMICAL INDUSTRIES LTD.
3-7-1, Kanda Nishiki-cho
Chiyoda-ku Tokyo(JP)

(71) Applicant: HODOGAYA CHEMICAL CO., LTD.
4-2, Toranomon 1 Chome Minato-ku
Tokyo(JP)

(72) Inventor: Ochiai, Yoshinori Seibutsu Kagaku K. Nissan Chem.
Ltd. 1470, Oaza Shiraoka Shiraoka-machi
Minami Saitama-gun Saitama-ken(JP)

(72) Inventor: Hanaue, Masami Seibutsu Kagaku K. Nissan Chem.
Ltd. 1470, Oaza Shiraoka Shiraoka-machi
Minami Saitama-gun Saitama-ken(JP)

(72) Inventor: Yamazaki, Mitsumasa c/o Chuo Kenkyusho
Hodogaya Chemical Co. Ltd. 6-2-30, Ouji
Kita-ku Tokyo(JP)

(72) Inventor: Kawada, Hiroshi c/o Chuo Kenkyusho
Hodogaya Chemical Co. Ltd. 6-2-30, Ouji
Kita-ku Tokyo(JP)

(72) Inventor: Hoya, Masaaki c/o Chuo Kenkyusho
Hodogaya Chemical Co. Ltd. 6-2-30, Ouji
Kita-ku Tokyo(JP)

(74) Representative: Patentanwälte Schaad, Balass & Partner
Dufourstrasse 101 Postfach
CH-8034 Zürich(CH)

## (54) Benzamide derivatives, process for producing the same, and soil fungicides containing the same.

(57) This invention relates to novel benzamide derivatives of the following general formula (I)

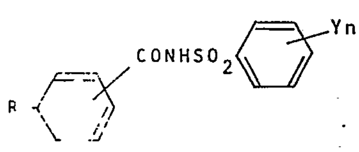

(I)

wherein R represents a radical

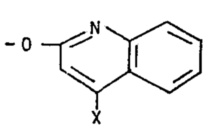

(in which X is hydrogen atom or a lower alkyl group) or a branched alkyl group, Y represents hydrogen atom, a halogen atom, a lower alkyl, a lower alkoxy, nitro, an alkoxycarbonyl or trifluoromethyl group, and n is an integer from 1 to 5, to a process for producing the same, and to soil fungicides containing the same as active ingredient.

EP 0 194 599 A2

# BENZAMIDE DERIVATIVES, PROCESS FOR PRODUCING THE SAME, AND SOIL FUNGICIDES CONTAINING THE SAME

## BACKGROUND OF THE INVENTION

( Field of the Invention )

This invention relates to novel benzamide derivatives, to a process for producing the same, to soil fungicides containing the same as active ingredient, and to a method of controlling soil borne diseases by using the same.

( Description of the Prior Art )

One of the important issues involved in agricultural production is the injuries by continuous cropping, which are known to be caused mainly by soil borne diseases. Controlling soil borne diseases is an extremely difficult task because of the ecology of their development, and there has been a strong demand for the development of powerful fungicides that can achieve this purpose. For example, clubroot which infests cruciferous vegetables has been spreading in recent years, inflicting a great blow to the production of cabbages, Chinese cabbages, Japanese turnips and other vegetables.

The area infested with rizomania, which is caused by beet necrotic yellow vein virus via Polymyxa betae (a soil microorganism) is also expanding, with the result that the damage to beet production is becoming increasingly heavier. Furthermore, the spread of potato scab has damaged potato production a great deal. Although

various types of fungicides have been applied to control these soil borne diseases, any of these fails to achieve outstanding effects. Same commercial fungicides are applied in order to control clubroot, but these commercial fungicides must be used in large quantities to achieve expected effects, but can cause problems of health hazards and environmental pollution if used in so much amounts.

The compounds represented by general formula (I) given below are new compounds that are not found in the literature, and the present inventors have found that these compounds have powerful fungicidal activity against soil borne diseases, particularly against the clubroot. This invention was accomplished based on these findings.

As known compounds which are close in structure to the compounds of this invention, there may be mentioned N-acyl-sulfonamides disclosed in U.S.P. No. 4,266,078 and N-phenyl-sulfonylbenzamides disclosed in Japanese Patent Application Laid-open No. 152,401/1985. Both of these differ from the compounds of this invention in the substituent groups involved. In addition, the former U.S. patent relates to herbicides, while the latter is concerned with fungicidal compositions for agricultural and horticultural use but gives no description on the control of soilborne diseases.

## SUMMARY OF THE INVENTION

An object of this invention is to provide benzamide



05. JUN 1986

derivatives of the following general formula (I)

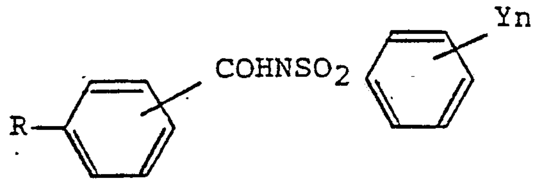

(I)

wherein R represents a radical 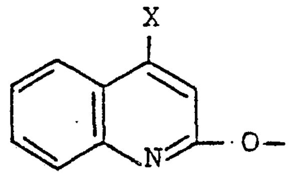 ( in which

X is hydrogen atom or a lower alkyl group ) or a branched alkyl group, Y represents hydrogen atom, a halogen atom, a lower alkyl, a lower alkoxy, nitro, an alkoxycarbonyl or trifluoromethyl group, and n is an integer from 1 to 5.

A further object of this invention is to provide a process for producing the compounds of the above general formula (I).

Another object of this invention is to provide fungicidal compositions containing, as active ingredient, at least one compound of the above general formula (I), particularly for the control of clubroot infecting cruciferous vegetables.

A still further object of this invention is to provide a method of controlling soil borne diseases by application of at least one compound of the above general formula.

## DETAILED DESCRIPTION OF THE PREFERRED EMBODIMENTS

The compounds of this invention will be described below in more detail.

In the above general formula (I), R is preferably



05. JUN 1986

a radical 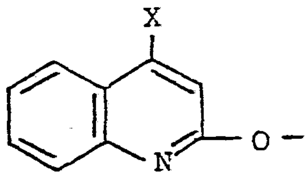 or a branched alkyl group having 3 to 6 carbon atoms; X is preferably hydrogen atom or an alkyl group having 1 to 3 carbon atoms; Y is preferably hydrogen atom, a halogen atom, an alkyl group having 1 to 6 carbon atoms, an alkoxy having 1 to 8 carbon atoms, nitro, an alkoxycarbonyl having 1 to 8 carbon atoms or trifluoromethyl group; and n is an integer from 1 to 5.

Most preferred substituent groups in the above general formula (I) to achieve high activity to control soil borne diseases are: a radical 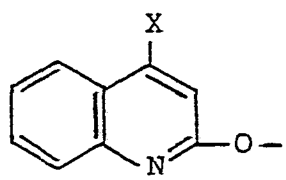 ( in which X is hydrogen atom or methyl group ) or a branched alkyl group having 3 to 4 carbon atoms ( e.g., iso-propyl or tert-butyl ) for R; and an alkyl group having 1 to 3 carbon atoms ( e.g., methyl, ethyl or iso-propyl ) or a halogen atom ( e.g., chlorine or bromine atom ) for Y.

Of the compounds of this invention which are listed below, compounds No.1, 3, 5, 7, 8, 9, 11, 12, 13, 24, 27, 37, 40, 45 and 47 are preferable in terms of activity against soil borne diseases, and compounds No. 1, 8, 11, 12, 13, 24, 37, 45 and 47 are the most preferred.

The compounds of this invention can be prepared by the reaction shown below.

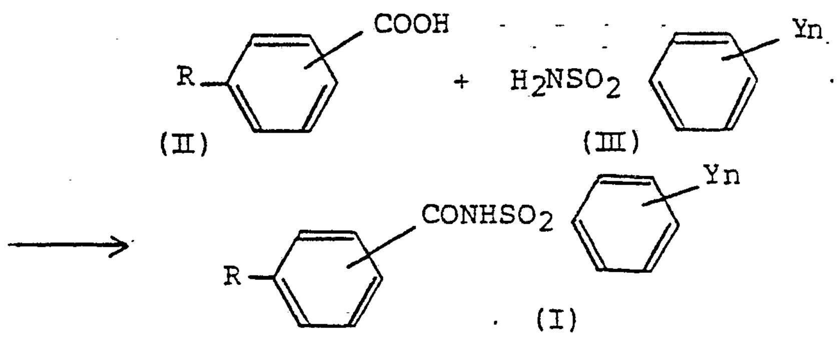

In the formulae (I) to (III) above, R, Y and n are as defined for formula (I); that is, by condensation of a substituted benzoic acid (II), or a reactive derivative thereof, with a benzenesulfonamide derivative (III). The reactive derivative of substituted benzoic acid (II) includes corresponding acid halides, acid anhydrides and esters, but use of the corresponding acid chloride is most common. In this case, the reaction is preferably carried out in the presence of a dehydrochlorinating agent. As typical examples of the dehydrochlorinating agent may be mentioned hydroxides such as sodium hydroxide and potassium hydroxide, carbonates such as sodium bicarbonate, sodium carbonate and potassium carbonate, and amines such as triethylamine and pyridine. The acid chloride can be obtained by reacting the corresponding substituted benzoic acid with thionyl chloride, phosgene or phosphorus oxychloride. The condensation reaction is preferably carried out in a solvent inert to the reaction. Typical examples of the inert solvents include aromatic hydrocarbons such as benzene,

toluene and xylene, ethers such as diethyl ether, tetrahydrofuran and dioxane, and polar solvents such as ethyl acetate, dimethylformamide and dimethylsulfoxide. These solvents may be used either alone or in combination. It is also possible to use the benzensulfonamide derivative (III) in the form of sodium or potassim salt in the condensation reaction, which has been previously prepared by reaction with sodium hydroxide, potassium hydroxide, sodium hydride or potassium hydride.

The condensation reaction may be conducted at any desired temperature in the range from room temperature to the boiling point of solvent used, but operation control is most easy if the reaction is carried out under reflux condition. Those skilled in the art will be able to set optimum reaction conditions for each case.

(Quinolin-2-yloxy)benzoic acid or a derivative thereof, which is an example of the starting materials, can be obtained by reaction of a 2-halogenoquinoline ( or a derivative thereof ) with a hydroxybenzoic acid ester to form (quinolin-2-yloxy)benzoic acid or a derivative thereof, followed by hydrolysis.

The following examples will further illustrate the process for producing the compounds of this invention, but are not intended to limit the scope of the invention.



05. JUN. 1986

( Example 1 )

Preparation of N-(2-methylbenzenesulfonyl)-3-(quinolin-2-yloxy)benzamide ( Compound No. 24 )

(1) Preparation of 3-(quinolin-2-yloxy)benzoic acid

32. 7 g (0.2 mole) of 2-Chloroquinoline and 33.2 g (0.2 mole ) of ethyl 3-hydroxybenzoate were dissolved in 200 ml of N,N-dimethylformamide, 55.3 g (0.4 mole) of potassium carbonate powder was added to the solution, and the mixture was heated at 140°C for six hours. After cooling, the reaction mixture was poured into 500 ml of water, and the oil which separated out was extracted with 200 ml of methylene chloride. The methylene chloride layer was washed with water and dried over anhydrous sodium sulfate, and the solvent was removed by distillation under reduced pressure. 100 ml of 20 % aqueous sodium hydroxide solution and 100 ml of ethanol were added to the residue, and the mixture was heated under reflux for one hour. After the reaction, 400 ml of water was added, the resulting mixture was neutralized and acidified to pH 3 to 4 by addition of hydrochloric acid, and the crystals obtained were collected by filtration and dried, giving 42.2 g of 3-(quinolin-2-yloxy)benzoic acid. Yield: 80.0%, m.p.: 165-167°C.

(2) Preparation of 3-(quinolin-2-yloxy)benzoyl chloride

26.5 g (0.1 mole) of 3-(quinolin-2-yloxy)benzoic acid obtained above was dissolved in 150 ml of dioxane and was added 35.7 g ( 0.3 mole ) of thionyl chloride. 0.5 ml of N,N-dimethylformamide as catalyst was further added thereto,



05. JUN. 1986

and the mixture was heated under reflux for three hours. After the reaction the dioxane and excess thionyl chloride were distilled off under reduced pressure. 200 ml of methylene chloride was added to the residue, the resulting mixture was washed with 200 ml of saturated aqueous solution of sodium bicarbonate and then with water, the methylene chloride layer was dried over anhydrous sodium sulfate, and the solvent was distilled off under reduced pressure, giving 27.0 g of 3-(quinolin-2-yloxy)-benzoyl chloride. Yield: 95.3%, m.p.: 117-120°C.

(3) Preparation of N-(2-methylbenzenesulfonyl)-3-(quinolin-2-yloxy)benzamide

0.88 g (0.022 mole) of 60% Sodium hydride was added to a solution of 3.42 g (0.02 mole) of 2-methylbenzenesulfonamide in 100 ml of tetrahydrofuran, and the mixture was heated under reflux for one hour. The resulting solution was cooled to below 10°C, 5.67 g (0.02 mole) of 3-(quinolin-2-yloxy)benzoyl chloride prepared above was added, and the mixture was heated under reflux for one hour. After cooling, the reaction mixture was poured into 300 ml of water, and the oil which separated out was extracted with 100 ml of methylene chloride. The methylene chloride layer was washed with water and dried over anhydrous sodium sulfate, and the solvent was removed by distillation under reduced pressure. Recrystallization of the residue from a mixed solvent consisting of 20 ml of ethanol and 60 ml of



05. JUNI 1986

toluene afforded 4.12 g of N-(2-methyl-benzenesulfonyl)-3-(quinolin-2-yloxy)benzamide. Yield: 49.3%, m.p.: 189.0-190.5°C.

| ( Elemental analysis ) | C% | H% | N% |
|---|---|---|---|
| Calcd. | 66.02 | 4.33 | 6.69 |
| Found | 65.85 | 4.38 | 6.55 |

( Example 2 )

Preparation of N-(5-chloro-2-methylbenzenesulfonyl)-3-(quinolin-2-yloxy)benzamide ( Compound No. 37 )

1.45 g (7.05 millimoles) of 5-Chloro-2-methylbenzenesulfonamide and 2.00 g (7.05 millimoles) of 3-(quinolin-2-yloxy)benzoyl chloride were dissolved in 20 ml dioxane, 1.30 g (9.41 millimoles) of potassium carbonate was added, and the mixture was heated under reflux for four hours. After the reaction the dioxane was distilled off under reduced pressure, 100 ml of water was added to the residue, and the insoluble matters were filtered off. The filtrate was neutralized and acidified to pH 3 to 4 by addition of dilute hydrochloric acid, and the crystals which separated out were collected by filtration and dried, giving 2.62 g of N-(5-chloro-2-methyl-benzenesulfonyl)-3-(quinolin-2-yloxy)benzamide. Yield: 82.1%, m.p.: 188-192°C.

| ( Elemental analysis ) | C% | H% | N% |
|---|---|---|---|
| Calcd. | 61.02 | 3.78 | 6.18 |
| Found | 60.83 | 3.85 | 6.31 |



05. JUN. 1986

( Example 3 )

Preparation of N-(2,4,6-triethylbenzenesulfonyl)-4-t-butylbenzamide ( Compound No. 9 )

0.42 g (0.0105 mole) of 60% Oily suspension of sodium hydride was placed in a 50-ml four-necked flask, and 15 ml of tetrahydrofuran dehydrated by usual method was added. 2.41 g (0.010 mole) of 2,4,6-triethylbenzenesulfonamide was then added in small portions, and the mixture was stirred for 20 minutes to form a solution of sodium salt of 2,4,6-triethylbenzenesulfonamide. To this was added dropwise a solution of 1.97 g (0.010 mole) of 4-t-butylbenzoyl chloride in 15 ml of dry tetrahydrofuran with stirring under ice cooling. After the dropwise addition the mixture was heated under reflux for four hours. The reaction mixture was poured into 400 ml of water, and hydrochloric acid was added thereto to adjust the solution to strong acidic of pH 1 to 2. After being saturated with sodium chloride, the aqueous solution was extracted thrice with each 100 ml of methylene chloride, and the combined organic solution was washed twice with each 100 ml of saturatd aqueous solution of sodium chloride and dried over anhydrous magnesium sulfate. The solvent was distilled off under reduced pressure, n-hexane was added to the residue, and the solidified product thus obtained was recrystallized from toluene, affording 2.12 g of N-(2,4,6-triethylbenzene-sulfonyl)-4-t-butylbenzamide. Yield: 52.9%, m.p.: 164-172°C.



05. JUI. 1986

| ( Elemental analysis ) | C% | H% | N% |
|---|---|---|---|
| Calcd. | 68.79 | 7.80 | 3.49 |
| Found | 68.62 | 7.71 | 3.63 |

( Example 4 )

Preparation of N-(2,4,6-triisopropylbenzenesulfonyl)-4-t-butylbenzamide ( Compound No. 12 )

8.50 g (0.030 mole) of 2,4,6-triisopropylbenzenesulfonamide, 5.40 g (0.039 mole) of potassium carbonate and 70 ml of dioxane were placed in a 200-ml four-necked flask, and a solution of 5.90 g (0.030 mole) of 4-t-butylbenzoyl chloride in 30 ml of dioxane was slowly added at room temperature with stirring. After the addition the mixture was heated under reflux of dioxane with stirring for six hours. After cooling, the insoluble matters were filtered off, the solid was washed with 30 ml of dioxane, the filtrate and the washings were combined together, and the combined solution was concentrated under reduced pressure. 200 ml of ethyl acetate was added to the residue, and 100 ml of ice water was added to the ethyl acetate solution, and hydrochloric acid was added with vigorous stiring until the pH of the aqueous layer fell down to 1 to 2. The ethyl acetate layer was collected, dried over anhydrous magnesium sulfate, and concentrated under reduced pressure. The residue was recrystallized from a 1:1 mixture of toluene and ethanol, affording 9.06 g of N-(2,4,6-triisopropylbenzenesulfonyl)-4-



05. JUN. 1986

t-butylbenzamide. Yield: 68.1%, m.p.: 204-206°C.

| ( Elemental analysis ) | C% | H% | N% |
|---|---|---|---|
| Calcd. | 68.79 | 7.80 | 3.49 |
| Found | 68.62 | 7.71 | 3.63 |

( Example 5 )

Preparation of N-(5-chloro-2-methylbenzenesulfonyl)-4-t-butylbenzamide ( Compound No. 13 )

6.17 g (0.030 mole) of 5-chloro-2-methylbenzenesulfonamide, 5.40 g (0.039 mole) of potassium carbonate and 70 ml of dioxane were placed in a 200-ml four-necked flask, and a solution of 5.90 g (0.030 mole) of 4-t-butylbenzoyl chloride in 30 ml of dioxane was portionwise added at room temperature with stirring. After the addition the mixture was then heated under reflux with stirring for eight hours. The reaction mixture, which turned into slurry upon cooling, was suction-filtered through a glass filter and the obtained residue was washed with a small amount of dioxane and dispersed in 1000 ml of water. Hydrochloric acid was added to the dispersion to adjust the solution to strong acidic of pH 1 to 2, and the precipitate thus formed was collected by filtration, washed with water and dried. Recrystallization from ethanol gave 8.46 g of N-(5-chloro-2-methylbenzenesulfonyl)-4-t-butylbenzamide. Yield: 77.1%, m.p.: 210-212°C.



05. JUN 1986

| ( Elemental analysis ) | C% | H% | N% |
|---|---|---|---|
| Calcd. | 59.08 | 5.52 | 3.83 |
| Found | 58.93 | 5.42 | 3.96 |

Some examples of the compounds of this invention are listed in Tables 1 and 2 below together with the compounds prepared in the above Examples. However, the compounds of this invention are not confined thereto.

05. JUNI 1986

Table 1

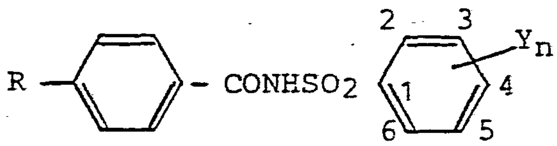

| Compound No. | R | Yn | Melting point (°C) |
|---|---|---|---|
| 1 | t-Bu | 2-Me | 213 - 215 |
| 2 | t-Bu | 4-Me | 203 - 205 |
| 3 | t-Bu | 2,5-$Me_2$ | 201 - 202 |
| 4 | t-Bu | 2,4,6-$Me_3$ | 220 - 221 |
| 5 | t-Bu | 2,3,5,6-$Me_4$ | 234 - 235 |
| 6 | t-Bu | 2,3,4,5,6-$Me_5$ | 204 - 205 |
| 7 | t-Bu | 4-Et | 187 - 188 |
| 8 | t-Bu | 2,5-$Et_2$ | 170 - 171 |
| 9 | t-Bu | 2,4,6-$Et_3$ | 164 - 172 |
| 10 | t-Bu | 4-OMe | 187 - 188 |
| 11 | t-Bu | 2,5-$(i\text{-}Pr)_2$ | 205 - 207 |
| 12 | t-Bu | 2,4,6-$(i\text{-}Pr)_3$ | 204 - 206 |
| 13 | t-Bu | 2-Me,5-Cl | 210 - 212 |
| 14 | t-Bu | 3,5-$(CF_3)_2$ | 161 - 166 |
| 15 | t-Bu | 4-Cl | 214 - 216 |
| 16 | t-Bu | 2,5-$Cl_2$ | 233 - 238 |
| 17 | t-Bu | 2,4,5-$Cl_3$ | 257 - 260 |
| 18 | t-Bu | 4-Br | 218 - 219 |
| 19 | t-Bu | 4-F | 187 - 200 |
| 20 | t-Bu | 2-$NO_2$ | 189 - 197 |
| 21 | t-Bu | 3-$NO_2$ | 233 - 235 |
| 22 | i-Pr | 2,4,6-$(i\text{-}Pr)_3$ | 198 - 200 |

**In Table 1, Me is methyl group, i-Pr is isopropyl group and t-Bu is tertiary-butyl group.

05. ... 1986

Table 2

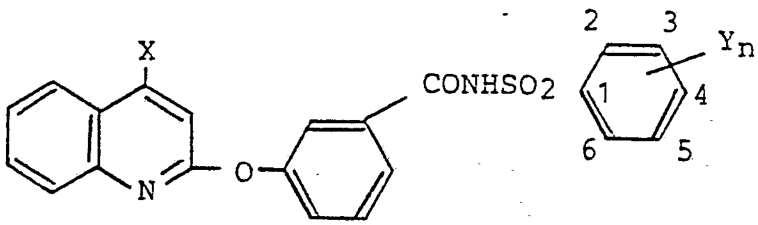

| Compound No. | X | Yn | Melting point (°C) |
|---|---|---|---|
| 23 | H | H | 188 - 191 |
| 24 | H | 2-Me | 189.0 - 190.5 |
| 25 | H | 4-Me | 181 - 184 |
| 26 | H | 4-OMe | 185 - 188 |
| 27 | H | 4-Et | 175 - 178 |
| 28 | H | 2-Cl | 181 - 184 |
| 29 | H | 4-Cl | 188 - 191 |
| 30 | H | 4-Br | 186 - 189 |
| 31 | H | 4-F | 182 - 185 |
| 32 | H | $3-NO_2$ | 190 - 192 |
| 33 | H | $4-NO_2$ | 195 - 198 |
| 34 | Me | H | 198 - 202 |
| 35 | Me | 2-Me | 181 - 185 |
| 36 | Me | 4-Me | 178 - 181 |
| 37 | H | 2-Me,5-Cl | 188 - 192 |
| 38 | H | 2-Me,4-Cl | 185 - 188 |
| 39 | H | $2,4-Me_2$ | 151 - 154 |
| 40 | H | $2,5-Me_2$ | 148 - 152 |
| 41 | H | $2,4-Cl_2$,5-COOMe | 225 - 228 |
| 42 | H | $2,5-Cl_2$ | 153 - 156 |
| 43 | H | $2,4,5-Cl_3$ | 145 - 149 |
| 44 | H | $2,4,6-Me_3$ | 100 - 104 |
| 45 | H | $2,4,6-(i-Pr)_3$ | 185 - 186 |
| 46 | H | $3,5-(CF_3)_2$ | 160 - 168 |
| 47 | H | $2,5-(i-Pr)_2$ | 195 - 200 |
| 48 | H | $2,3,4,5,6-Me_5$ | 210 - 211 |

05. JUN 1986

Table 2 (Cont.)

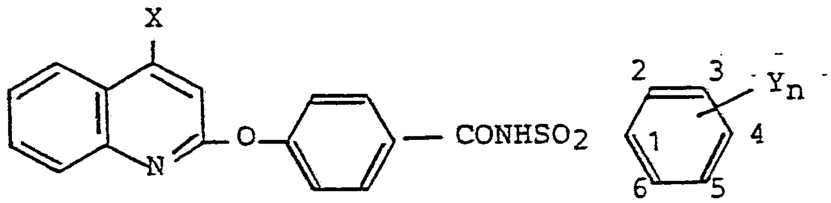

| Compound No. | X | Yn | m.p. (°C) |
|---|---|---|---|
| 49 | H | H | 183 - 186 |
| 50 | H | 2-Me | 175 - 178 |
| 51 | H | 4-Me | 186 - 189 |
| 52 | Me | H | 191 - 194 |
| 53 | Me | 2-Me | 205 - 208 |
| 54 | Me | 4-Me | 197 - 200 |

**In Table 2, Me is methyl group, Et is ethyl group and i-Pr is isopropyl group.



05. JUN 1986

In actual application as soil fungicide, the compounds of this invention may be used in combination with a suitable carrier, including solid carriers such as clay, talc, bentonite, diatomaceous earth and calcium carbonate, and liquid carriers such as water, alcohols ( e.g., methanol and ethanol ), aromatic hydrocarbons ( e.g., benzene, toluene and xylene ), chlorinated hydrocarbons, ethers, ketones, esters( e.g., ethyl acetate ) and acid amides ( e.g., dimethylformamide ). Emulsifiers, dispersants, suspending agents, penetrating agents, spreading agents, stabilizers, auxiliary substance and other additives may further be added as required to prepare desired types of formulation, such as liquid formulation, emulsifiable concentrate, wettable powder, dust and granules.

There is no specific limitation upon the content of the compound of this invention when it is applied in the form of solid ( dust, granules or wettable powder ), but the preferable level is in the range from about 0.1 to 60 weight %, most preferably in the range from about 0.5 to 30 weight %.

No specific limitation also exists when it is applied in the form of liquid ( emulsifiable concentrate or liquid ), but the preferable level is in the range from about 0.5 to 90 weight %, most preferably in the range from about 2 to 80 weight %. It is preferable that the fungicidal composition of this invention is directly applied onto soil

and mixed together with soil in the case of dust or granules. There is no specific limitation upon the amount of these compounds of the present invention, but the suitable level is in the range from about 0.1 to 200 Kg/ha, most preferably in the range from about 0.5 to 100 Kg/ha.

When the fungicidal composition is an emulsifiable concentrate, liquid formulation or wettable powder, it is diluted with water before use to a prescribed concentration and the soil being treated is drenched with this resulted aqueous formulation. No specific limitation also exists upon the concentration of the compound of this invention and the amount of liquid to be applied, but the suitable level is in the range from about 1 to 2000 ppm, most preferably in the range from about 50 to 1000 ppm, and in the range from about 1 to 200 Kg/ha, most preferably in the range from about 5 to 100 Kg/ha, respectively.

Of the various types of formulation mentioned above, dust is the most preferred. Any solid carried may be used in this case, but clay, talc, calcium carbonate and diatomaceous earth are employed most advantageously. These may be used either alone or in combination. In addition, white carbon may also be added as an auxiliary substance if required. The suitable proportion of these components is 1 to 20 weight parts of a compound of this invention, 60 to 99 weight parts of clay, talc, calcium carbonate or diatomaceous earth, and 0 to 20 weight parts of white carbon (



05. JUN 1986

auxiliary substance ).

The soil borne diseases that can be controlled by the method of this invention include, among others, clubroot, rizomania and potato scab.

Formulation examples of fungicidal composition containing the compound of this invention as active ingredient will be described below, which are offerred by way of illustration and not by way of limitation. The "parts" in these examples are all by weight.

( Formulation Example 1 ) Wettable powder

| | |
|---|---|
| Compound of this invention | 25 parts |
| Siegreit A<br>( trade name: Kaolin clay manufactured by Siegreit Mining Industries Co., Ltd.) | 69 parts |
| Solpol 5039<br>( trade name: mixture of nonionic and anionic surface-active agents manufactured by Toho Chemical Co., Ltd. ) | 3 parts |
| Carplex ( antiblocking agent )<br>( trade name: White carbon manufactured by Shionogi Seiyaku K.K. ) | 3 parts |

The above components are homogeneously mixed together and ground to form a wettable powder. Upon use, the wettable powder is diluted with water up to one two hundred and fiftieth to one twenty-five thousandth.



05. JUN. 1986

( Formulation Example 2 ) Emulsifiable concentrate

| | |
|---|---|
| Compound of this invention | 50 parts |
| Xylene | 25 parts |
| Dimethylformamide | 20 parts |
| Solpol 2680 | 5 parts |

( trade name: mixture of nonionic and anionic surface-active manufactured by Toho Chemical Co., Ltd. )

The above components are mixed intimately together to form an emulsifiable concentrate. Upon use, the emulsifiable concentrate is diluted with water up to one five hundredth to one fifty thousandth in concentration.

( Formulation Example 3 ) Dust

| | |
|---|---|
| Compound of this invention | 5 parts |
| Clay | 95 parts |

The above components are homogeneously mixed to give dust containing 5% active ingredient.

( Formulation Example 4 ) Dust

| | |
|---|---|
| Compound of this invention | 1 parts |
| Talc | 99 parts |

The above components are homogeneously mixed to give dust containing 1% active ingredient.

( Formulation Example 5 ) Dust

| | |
|---|---|
| Compound of this invention | 20 parts |
| Clay | 70 parts |
| White carbon | 10 parts |

The above components are homogeneously mixed to give dust containing 20% active ingredient.

( Formulation Example 6 ) Granules

| | |
|---|---|
| Compound of this invention | 5 parts |
| Bentonite | 25 parts |
| Talc | 70 parts |

The above components are mixed intimately together and ground, incorporated with a small amount of water and mixed together with stirring. The resulting mixture is granulated by means of extrusion-granulator and dried to form granules.

The efficacy of the compounds of this invention as soil fungicide will become apparent from the following test examples.

( Test Example 1 )

Test on clubroot ( emulsion treatment ):

Sterilized soil was filled in plastic pots (8 cm in diameter), three seeds of Chinese cabbage ( variety: "Kinshoh" No.2 ) were sown in each pot, and 10 ml of fungicide emulsion ( prepared by diluting the emulsifiable concentrate of Formulation Example 2 by a prescribed factor ) was applied to the surface of soil in each pot. On the

next day, a resting spore suspension ( prepared by homogenizing 1 g of swelling roots of Chinese cabbage caused by infection of Plasmodiophora brassicae ) was inoculated to the surface of soil in each pot by drenching. The pots were placed in a greenhouse to grow Chinese cabbages. On the five weeks after the inoculation, the existence and size of swollen root were observed, and the protective value of each fungicide was calculated according to the formula given below.

$$\text{Disease Index} = ( 0 \times n_0 + 1 \times n_1 + 2 \times n_2 + 3 \times n_3)/N$$

$n_0$: Number of normal seedlings

$n_1$: Number of seedlings in which only branch roots are affected.

$n_2$: Number of seedlings in which both the main and branch roots are slightly affected.

$n_3$: Number of seedlings in which both the main and branch roots are heavily affected.

N : Total number of seedlings tested.

$$\text{Protective Value} = (1 - \frac{\text{Disease Index of Treated Pots}}{\text{Disease Index of Control Pots}}) \times 100$$

PCNB wettable powder ( active ingredient: 75% ) was used as comparative fungicide in this test.

The test result is summarized in Table 3.

05 JUN. 1986

Table 3

| Compound | Concentration of active ingredient (ppm) | Protective Value |
|---|---|---|
| No. 1 | 500 | 85 |
| | 100 | 80 |
| No. 2 | 100 | 100 |
| No. 3 | 500 | 100 |
| | 100 | 100 |
| No. 4 | 500 | 100 |
| | 100 | 100 |
| No. 5 | 500 | 100 |
| | 100 | 100 |
| No. 6 | 500 | 100 |
| | 100 | 90 |
| No. 7 | 100 | 100 |
| No. 8 | 500 | 100 |
| | 100 | 100 |
| No. 9 | 500 | 100 |
| | 100 | 100 |
| No. 10 | 500 | 93 |
| | 100 | 90 |
| No. 11 | 500 | 100 |
| | 100 | 100 |
| No. 12 | 500 | 100 |
| | 100 | 100 |
| No. 13 | 500 | 100 |
| | 100 | 100 |
| No. 14 | 500 | 99 |
| | 100 | 97 |
| No. 15 | 100 | 100 |

05. JUN 1986

Table 3 (cont.)

| Compound | Concentration of active ingredient (ppm) | Protective Value |
|---|---|---|
| No. 16 | 100 | 100 |
| No. 17 | 500 | 100 |
| | 100 | 80 |
| No. 18 | 100 | 100 |
| No. 19 | 500 | 100 |
| | 100 | 100 |
| No. 20 | 500 | 100 |
| No. 21 | 500 | 100 |
| No. 23 | 500 | 85 |
| No. 24 | 500 | 100 |
| | 100 | 100 |
| No. 25 | 500 | 100 |
| | 100 | 70 |
| No. 26 | 500 | 100 |
| | 100 | 60 |
| No. 27 | 500 | 100 |
| | 100 | 85 |
| No. 28 | 500 | 100 |
| | 100 | 100 |
| No. 29 | 500 | 100 |
| | 100 | 65 |
| No. 30 | 500 | 100 |
| No. 31 | 500 | 100 |
| No. 32 | 500 | 93 |
| No. 34 | 500 | 96 |
| No. 35 | 500 | 100 |
| | 100 | 60 |
| No. 36 | 500 | 100 |
| | 100 | 80 |

Table 3 (cont.)

| Compound | Concentration of active ingredient (ppm) | Protective Value |
|---|---|---|
| No. 37 | 500 | 100 |
| | 100 | 100 |
| No. 38 | 500 | 100 |
| | 100 | 70 |
| No. 39 | 500 | 100 |
| | 100 | 100 |
| No. 45 | 500 | 100 |
| | 100 | 100 |
| No. 46 | 500 | 100 |
| | 100 | 60 |
| No. 47 | 500 | 100 |
| | 100 | 70 |
| No. 48 | 500 | 100 |
| No. 49 | 500 | 100 |
| No. 50 | 500 | 100 |
| | 100 | 100 |
| No. 51 | 500 | 100 |
| No. 52 | 500 | 100 |
| No. 53 | 500 | 100 |
| No. 54 | 500 | 80 |
| PCNB | 500 | 32 |
| | 100 | 0 |

**In Table 3, PCNB is pentachloronitrobenzene which is a commercial soil fungicide having the following structure:

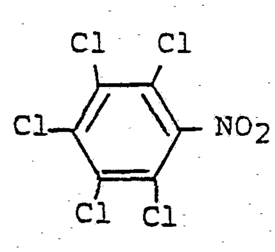

PCNB

( Test Example 2 )

Test on clubroot ( dust treatment ):

180 g of Sterilized soil was mixed well with 50 mg of dust containing the compound to be tested of this invention in concentration of 5 %. ( application dosage of active ingredient (a.i.): 5kg/hectare ) The mixed soil thus prepared was filled in are/20,000 pot, and three seeds of Chinese cabbage ( variety: "Kinshoh" No.2 ) were sown in each pot. On the next day, a resting spore suspension ( prepared by homogenizing 1 g of swollen root of Chinese cabbage caused by infection of Plasmodiophora brassicae ) was inoculated to the surface of soil in each pot by drenching. The pots were placed in a greenhouse to grow cabbages. On the five weeks after the inoculation, the existence and size of swollen root were observed, and the protective value of each fungicide was calculated in the same way as in Test Example 1.

500 mg of PCNB dust ( active ingredient: 20% ) was used as comparative fungicide in this test.

The test result is summarized in Table 4.

05. JUN. 1986

Table 4

| Compound No. | Application dosage (a.i. Kg/hectare) | Protective Value |
|---|---|---|
| 1 | 5 | 100 |
| 2 | 5 | 100 |
| 3 | 5 | 100 |
| 4 | 5 | 100 |
| 5 | 5 | 100 |
| 7 | 5 | 100 |
| 8 | 5 | 100 |
| 9 | 5 | 100 |
| 11 | 5 | 100 |
| 12 | 5 | 100 |
| 13 | 5 | 100 |
| 15 | 5 | 100 |
| 16 | 5 | 100 |
| 18 | 5 | 100 |
| 19 | 5 | 100 |
| 24 | 5 | 100 |
| 27 | 5 | 90 |
| 28 | 5 | 100 |
| 31 | 5 | 100 |
| 35 | 5 | 100 |
| 37 | 5 | 100 |
| 39 | 5 | 100 |
| 45 | 5 | 100 |
| 47 | 5 | 90 |
| PCNB | 20 | 70 |

05. JUN. 1986

WHAT IS CLAIMED IS:

1. A benzamide derivative of the general formula (I)

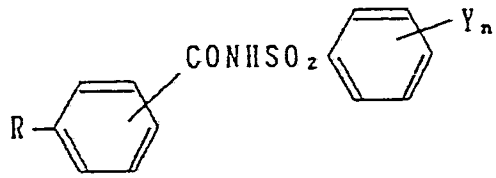

(I)

wherein R represents a radical 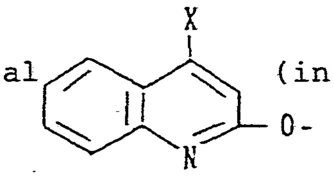 (in which X is hydrogen atom or a lower alky group) or a branched alkyl group, Y represents hydrogen atom, a halogen atom, a lower alkyl, a lower alkoxy, nitro, an alkoxycarbonyl or trifluoromethyl group, and n is an integer from 1 to 5.

2. A benzamide derivative according to claim 1 of the formula (I), wherein R is 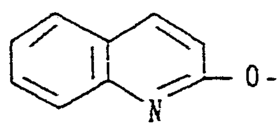 or t-butyl group, Y is an alkyl group of 1 to 3 carbon atoms or chlorine atom, and n is an integer from 1 to 3.

3. A process for preparing a benzamide derivative of the general formula (I)

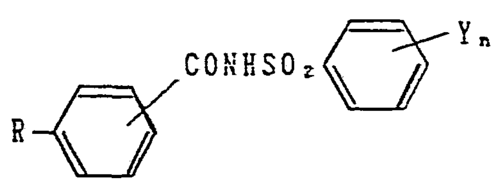

(I)

(wherein R, Y and n are as defined below, which comprises reacting a compound of the general formula (II)

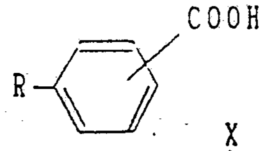

(II)

wherein R represents a radical 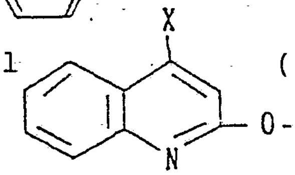 (in which X is hydrogen atom or a lower alkyl group) or a branched alkyl group or a reactive derivative thereof, with a sulfonamide derivative of the following general formula (III)

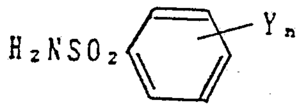

wherein Y represents hydrogen atom, a halogen atom, a lower alkyl, a lower alkoxy, nitro, an alkoxycarbonyl or trifluoromethyl group, and n is an integer from 1 to 5 .

4. Soil fungicide compositions containing, together with a suitable carrier or auxiliary substance, effective amounts of at least one benzamide derivative of the following general formula (I)

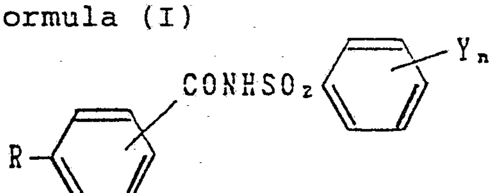

(I)

wherein R represents a radical 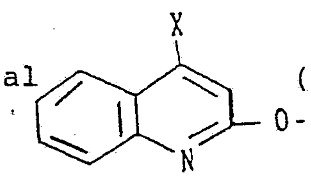 (in which X is hydrogen atom or a lower alkyl group) or a branched alkyl group, Y represents hydrogen atom, a halogen atom, a lower alkyl, a lower alkoxy, nitro,

an alkoxycarbonyl ortrifluoromethyl group, and n is an integer from 1 to 5.

5. Soil fungicide compositions as defined in claim 3 for the controlling clubroot infecting cruciferous vegetable.

6. Soil fungicide compositions as defined in claim 3, wherein said soil fungicide compositions contain, as active ingredient a compound having the formula (I) wherein R is 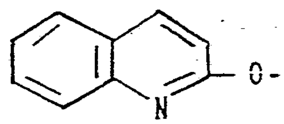 or t-butyl group, Y is an alkyl group of 1 to 3 carbon atoms or chlorine atom, and n is an integer from 1 to 3.